# EUROPEAN PATENT APPLICATION

(11) **EP 1 716 855 A1**
(43) Date of publication of application: **02.11.2006**
(21) Application number: 04711489.7
(22) Date of filing: 16.02.2004
(51) Int. Cl.: A61K 31/616, A61K 9/06, A61K 9/70, A61P 17/00, A61P 17/02, A61P 17/04, A61P 29/00, A61P 31/12, A61P 31/22

(54) **REMEDY FOR EXTERNAL USE FOR SKIN AND MUCOSAL INJURIES CAUSED BY VIRAL INFECTION**

(71) Applicant: TEIKOKU SEIYAKU CO., LTD., Higashikagawa-shi, Kagawa 769-2695 (JP)
(72) Inventor: INAMOTO, Yukiko 3372-18, Asano, Kagawa-ken (JP); KAWADA, Mitsuhiro, 7692702 (JP)
(74) Representative: Albrecht, Thomas
(86) International application number: PCT/JP2004/001617
(87) International publication number: WO 2005/077379

(57) **Abstract**

An external preparation containing acetylsalicylic acid or a pharmacologically acceptable salt thereof as an active ingredient, which is used for treatment of vesicle, sore or ulcer of skin or mucosa caused by a viral infection.

## Description

### TECHNICAL FIELD

The present invention relates to an excellent external preparation containing acetylsalicylic acid as an active ingredient, which has a therapeutic effect on skin or mucosal injury symptoms, such as vesicle, sore or ulcer caused by a viral infection such as varicella-zostervirus, herpes simplex virus or enterovirus together with an inhibition effect on a pain and pruritus at these affected or injured parts, and methods for treatment of these symptoms thereby.

### BACKGROUND ART

Although skin or mucosal injury symptoms caused by a viral infection such as varicella·zostervirus, herpes simplex virus or enterovirus are often seen, specific therapeutic methods on these symptoms have not been established. Now, in order to prevent the secondary infection, an antibiotic is administered, or disinfection at the affected parts by povidone iodine, etc. is carried out.

It is present status that there is hardly any external preparation which improves a viral infection injury symptom such as vesicle, sore or ulcer caused by a viral infection such as varicella·zostervirus, herpes simplex virus or enterovirus, and simultaneously inhibits a pain and pruritus at these affected parts.

Recently anti-viral agents have been developed, but these agents are hardly said to be satisfied with improvement in the viral infection injury symptoms such as vesicle, sore or ulcer, especially in inhibition on a pain and pruritus at these affected parts.

Furthermore, the effect of an external preparation containing a nonsteroidal anti-inflammatory agent or a steroid on the symptoms not satisfactory and that there are anxious about side effects thereby, such as local irritation-feeling and rubor.

By the way, acetylsalicylic acid (it may be called hereinafter aspirin) is mainly and widely used in the form of oral administration as an analgesic antipyretic for many years, owing to its powerful analgesic, antipyretic and anti-rheumatism activities, and is a medicine with high safety and with few side effects.

In recent years, the research on application to external preparations of acetylsalicylic acid is advanced.

Ointments for treatment of neuralgia in Japanese Patent Publication A 3-72426, external preparations for skin injury in Japanese Patent Publication A 9-235232, dermal administration system for treatment of anti-thrombus and for prevention of cancer in Japanese Patent Publication (Toku Hyo Hei) 8-504198, external preparations for treatment of allergic dermatitis in Japanese Patent Publication A 2001-187739, external preparations for antipruritus in WO 01/47525, etc. are reported, respectively.

However, there is no report on external preparations containing acetylsalicylic acid which are used in aiming to treat a viral infection injury such as vesicle, sore or ulcer caused by a viral infection such as varicella-zostervirus, herpes simplex virus or enterovirus, and to inhibit a pain or pruritus on these affected parts.

### DISCLOSURE OF INVENTION

The present invention is to solve the above problems, and its object is to provide an excellent external preparation containing acetylsalicylic acid as an active ingredient, which has few side effects, has a therapeutic effect on a viral infection injury symptom such as vesicle, sore or ulcer caused by a viral infection such as varicella· zostervirus, herpes simplex virus or enterovirus together with an inhibition effect on a pain and pruritus at these affected parts.

The present inventors have extensively studied to solve the above problems and found that an excellent external preparation containing acetylsalicylic acid as an active ingredient has few side effects, has an excellent therapeutic effect on a viral infection injury symptom such as vesicle, sore or ulcer caused by a viral infection such as varicella· zostervirus, herpes simplex virus or enterovirus together with a superior inhibition effect on a pain or pruritus at these affected parts.

In addition, even if the external preparation containing acetylsalicylic acid is applied to a pain and pruritus at the affected parts of the viral infection injury symptoms such as vesicle, sore or ulcer caused by a viral infection such as varicella-zostervirus, herpes simplex virus or enterovirus, any retardation of healing of the viral infection injury symptoms, such as vesicle, sore, and ulcer was not seen.

Furthermore, although this activity and effect depends on the concentration of acetylsalicylic acid in a preparation, the activity and effect hardly changes even in excess of a certain fixed concentration of acetylsalicylic acid.

### BEST MODE FOR CARRYING OUT THE INVENTION

The present invention relates to an excellent external preparation containing acetylsalicylic acid or a pharmacologically acceptable salt thereof as an active ingredient, which is used for treatment of the viral infection injury symptoms caused by a viral infection such as varicella· zostervirus, herpes simplex virus or enterovirus together with an inhibition effect on a pain and pruritus at these affected parts.

The present invention also relates to an excellent external preparation containing acetylsalicylic acid, or a pharmacologically acceptable salt thereof as an active ingredient, which is used for treatment of vesicle, sore or ulcer of skin and mucosa caused by a viral infection together with an inhibition effect on a pain and pruritus at these affected parts.

The present invention also relates to an excellent external preparation containing acetylsalicylic acid, or a pharmacologically acceptable salt thereof as an active ingredient, which is used for treatment of a pain and pruritus at the affected parts on the viral infection injury symptoms caused by a viral infection.

The present invention also relates to an excellent external preparation containing acetylsalicylic acid, or a pharmacologically acceptable salt thereof as an active ingredient, which is used for treatment of a pain and pruritus at the parts of vesicle, sore or ulcer of skin and mucosa caused by virus infection such as valicella·zostervirus, herpes simplex virus or enterovirus.

The present invention relates to also a method for treatment of skin or mucosal injury caused by a viral infection by administering an effective amount of acetylsalicylic acid or a pharmacologically acceptable salt thereof to an affected part of a patient.

The present invention relates to a method for treatment of vesicle, sore, or ulcer of skin or mucosa caused by a viral infections such as varicella·zostervirus, herpes simplex virus or enterovirus, administering an effective amount of an external preparation containing acetylsalicylic acid or a pharmacologically acceptable salt thereof to an affected part of a patient.

Furthermore, the present invention relates to a method for treatment of a pain and pruritus at skin and mucosal injury caused by a viral infection such as varicella-zostervirus, herpes simplex virus or enterovirus, administering an effective amount of an external preparation containing acetylsalicylic acid or a pharmacologically acceptable salt thereof to an affected part of a patient.

Acetylsalicylic acid contained in the external preparations of the present invention is listed in the Japanese Pharmacopoeia. The content of acetylsalicylic acid contained in the external preparations changes in accordance with forms of the preparation, and it is 0.05 to 80 % by the total weight for exhibiting a sufficient effect, preferably 0.05 to 70 % by the weight, and more preferably 0.01 to 50% by the weight.

When the content of acetylsalicylic acid is more 80 % by the weight, its physical property is hardly preserved, and when the content is less than 0.01 % by the weight, the activity of acetylsalicylic acid is not fully exhibited.

Acetylsalicylic acid and its pharmacologically acceptable salt such as a salt formed with an amino acid, such as DL-lysine, or a mineral salt such as sodium salt can be used as the active ingredient contained in the external preparations of the present invention.

Especially if the external preparations of the present invention are such a dosage form as an active ingredient is administered directly to local surface on skin, they will not be limited, and for example, ointments, creams, gels, solutions (suspensions, emulsions, lotions, etc.), cataplasms, tapes, aerosols, and powders for external use, can be used.

All can be used as far as they are an ingredient used for the usual external preparations as an ingredient for the external preparations containing acetylsalicylic acid of the present invention.

In case of ointments, creams, gels, solutions, suspensions, emulsions and lotions, bases, such as white petrolatum, yellow petrolatum, lanolin, white beeswax, cetyl alchohol, stearyl alcohol, stearic acid, hydrogenated oil, hydrocarbon gel, polyethylene glycols, liquid paraffin and squalane; solvents and solubilizing agents, such as oleic acid, isopropyl myristate, diisopropyl adipate, isopropyl sebacate, glyceryl triisooctanoate, crotamiton, diethyl sebacate, hexyl laurate, a fatty acid, a fatty acid ester, an aliphatic alcohol, a vegetable oil and an alcohol; antioxidants, such as a tocopherol derivative, L-ascorbic acid, dibutylhydroxytoluene, and butylated hydroxyanisole; antiseptics such as p-hydroxybenzoate; humectants, such as glycerin, propylene glycol, and hyaluronate sodium; surface active agents, such as polyoxyethylene derivative, glycerol ester of a fatty acid, sucrose ester of a fatty acid, sorbitan ester of a fatty acid, propylene glycol of a fatty acid, and lecithin; thickening agents, such as carboxy vinyl polymer, xanthane gum, carboxymethyl cellulose, carboxymethylcellulose sodium, hydroxypropylcellulose, and hydroxypropyl methylcellulose; stabilizers; preservatives; absorption enhancers, and other suitable excipients can be blended therein.

In case of cataplasms, tackifiers, such as polyacrylic acid and polyacrylic acid copolymer; crosslinking agents, such as aluminium sulfate, aluminium potassium sulfate, aluminium chloride, magnesium aluminometasilicate and dihydroxy aluminium acetate; thickening agents, such as sodium polyacrylate, polyvinyl alcohol, polyvinylpyrrolidone, gelatin, sodium alginate, carboxymethyl cellulose, carboxymethylcellulose sodium, hydroxypropylcellulose and hydroxypropyl methylcellulose; polyhydric alcohols such as glycerin, propylene glycol, and 1,3-butanediol; solvents and solubilizing agents, such as oleic acid, isopropyl myristate, diisopropyl adipate, isopropyl sebacate, glyceryl triisooctanoate, crotamiton, diethyl sebacate, hexyl laurate, a fatty acid, a fatty acid ester, an aliphatic alcohol, a vegetable oil and an alcohol; surface active agents such as a polyoxyethylene derivative; flavors such as 1-menthol; antiseptics such as p-hydroxybenzoate; purified water; and other suitable excipients can be blended therein.

In case of tapes, adhesives, such as styrene isoprene styrene block copolymer and an acrylic resin; tackifiers, such as alicyclic saturated-hydrocarbon resin, rosin resin, and terpene resin; softeners, such as liquid rubber and liquid paraffin; antioxidants such as dibutylhydroxytoluene; polyhydric alcohols such as propylene glycol; solvents and solubilizing agents, such as oleic acid, isopropyl myristate, diisopropyl adipate, isopropyl sebacate, glyceryl triisooctanoate, crotamiton, diethyl sebacate, hexyl laurate, a fatty acid, a fatty acid ester, an aliphatic alcohol, a vegetable oil and an alcohol; surface active agents such as a polyoxyethylene derivative; absorption enhancers and other suitable excipients can be blended therein. Moreover, by adding a polymer which can contain water such as sodium polyacrylate or polyvinyl alcohol and a small amount of purified water can be prepared aqueous tapes.

In case of external powders, vehicles, such as potato starch, rice starch, corn starch, talc, and zinc oxide, and other suitable excipients can be blended therein.

In case of aerosols, excipients used in ointments, creams, gels, solutions, emulsions, suspensions, lotions, external powders, etc., namely bases, such as white petrolatum, yellow petrolatum, lanolin, white beeswax, cetyl alcohol, stearyl alcohol, stearic acid, hydrogenated oil, hydrocarbon gel, polyethylene glycol, liquid paraffin and squalane; solvents and solubilizing agents, such as oleic acid, isopropyl myristate, diisopropyl adipate, diisopropyl sebacate, glyceryl triisooctanoate, crotamiton, diethyl sebacate, hexyl laurate, a fatty acid, a fatty acid ester, an aliphatic alcohol, a vegetable oil and an alcohol; antioxidants, such as a tocopherol derivative, L-ascorbic acid, dibutylhydroxytoluene, and butylated hydroxyanisole; antiseptics such as p-hydroxybenzoate; humectants, such as glycerin, propylene glycol, and hyaluronate sodium; surface active agents, such as a polyoxyethylene derivative, glycerol ester of a fatty acid, sucrose ester of a fatty acid, sorbitan ester of a fatty acid, propylene glycol of a fatty acid, and lecithin; stabilizers such as thickening agents, such as carboxy vinyl polymer, xanthane gum, carboxymethyl cellulose, carboxymethylcellulose sodium, hydroxypropylcellulose, and hydroxypropyl methylcellulose; vehicles, such as potato starch, rice starch, corn starch, talc, and zinc oxide; propellants, such as liquefied petroleum gas, liquefied carbon dioxide, dimethyl ether, nitrogen, kerosene, and carbon dioxide; buffers; correctives, suspending agents, emulsifiers, perfumes, preservatives, and other suitable excipients can be blended therein.

The external preparations of the present invention are manufactured using the conventional procedure for external preparations such as well blending each component and if necessary a base. They are used by applying them in usual methods to the affected region directly, or they are suspended on or immersed in cloth, etc. to apply them to the affected region.

In order to prepare ointments, by using fat, fatty oil, lanolin, wax, resin, plastics, glycol, a higher alcohol, glycerin, water, an emulsifier, a suspending agent or other suitable excipient as a raw material, or by using these materials as a base, an active ingredient is added thereto, and the mixture is homogenously blended to prepare ointments. The base is melted by heating to mix uniformly, and if necessary an additive, such as an absorption enhancer, an antioxidant, an antiseptic, a surface active agent or purified water is added thereto, and further fine powders of the active ingredient are blended with it to prepare ointments or creams.

For example, in order to prepare oleaginous ointments, after melting by warming a base, mixing and cooling halfway, the active ingredients other than the base which are liquefied or made fine powders are mixed with part of the base, and the base remaining is added thereto. The resulting mixture is kneaded together until all parts become homogenous.

For example, in order to prepare emulsion-ointments and water soluble ointments, after a solid base being melted on a water bath, it is kept at about 75 °C , and water in which a water-soluble base is dissolved, is warmed to this temperature or a little higher temperature, is added thereto. Then the mixture was homogenously blended to prepare them.

In order to prepare cataplasms, the active ingredients are previously mixed with an ointment base mainly containing a water soluble polymer which is rich in water retention, such as gelatin, carmellose sodium, methylcellulose, and sodium polyacrylate, and the mixture was expanded on a support such as an unwoven fabric, a surface of the base is covered with a plastic film, such as polyethylene or polypropylene, and it is cut in a desired size to prepare cataplasms.

In order to prepare tapes, to adhesives such as acrylic resin, or styrene isoprene styrene block copolymers are added tackfiers such as alicyclic saturated-hydrocarbon resin, rosin resin and terpene resin, softeners such as liquid rubber and liquid paraffin, absorption enhancers, an antioxidant, etc., and the mixture is dissolved in an organic solvent, such as toluene. The mixture was blended or was melted under heating and blended, and thereto were added liquefied or powdered active ingredients. The mixture was expanded on a release paper, and when the tape is a soluble type, after expanding and drying, it is laminated with a flexible support, such as a polyurethane film, a polyethylene film, a polyvinyl chloride film, a woven fabric, and an unwoven fabric, and it is cut in a desired size to prepare tapes.

In order to prepare lotions, an active ingredient, a solvent, an emulsifier, a suspending agent, etc. are added to an aqueous liquid, and the mixture is made homogenous. In regard to suspension-lotions, after an active ingredient is pulverized and is made easy to wet in water by glycerin or ethanol, a solution of a suspending agent or a lotion base is gradually added thereto, and the mixture is homogenously blended to prepare suspension-lotions. In regard to emulsion-lotions, an oil-soluble drug and an oil phase are put into one container, and the aqueous phase is put into the other container, and both containers are warmed. In case of making an O/W emulsion, an oil phase is gradually added to an aqueous phase, and in case of making a W/O emulsion, an aqueous phase is gradually added to an oil phase on the contrary, and the mixture continues mixing until emulsification is completely homogenized and serves as a homogeneous liquid.

In order to prepare external powders, an active ingredient, an additive and excipients such as potato starch, rice starch, corn starch, talc, and zinc oxide, are uniformly dispersed.

In order to prepare aerosols, solutions containing an active ingredient, ointments, creams, gels, suspensions, emulsions, solutions, lotions, external powders, etc. are prepared in accordance of the above mentioned methods and they are filled into a well-closed container with liquefied gas or compressed gas.

As a disease which is the treatment target of the external preparation of the present invention, for example, pandemic herpes, such as a eczema herpeticum, neonate pandemic herpes, and fetus herpes; areata herpes, such as herpes of labial and face, herpetic stomatitis, herpes genitalis, herpetic panaris and inoculation nature herpes; varicella or zoster virus, such as hand-foot-mouth disease, infectious disease by enterovirus (except for hand-foot-mouth disease); skin or mucosal injury syndromes, such as vesicle by infection of herpes simplex virus, enterovirus, etc., sore, or ulcer; local pain or pruritus at these affected parts, are illustrated.

Hereafter, although by illustrating examples and test examples on external preparations of this invention containing acetylsalicylic acid, the present invention should not be limited to these examples.

### Examples 1 to 2 (ointments)

According to the formulation shown in Table 1, acetylsalicylic acid was added to a mixture of a base and a solvent, and the mixture was well kneaded under stirring to prepare ointments.

**Table 1**

| Formulation of ointment containing acetylsalicylic acid | | |
|---|---|---|
| Example | 1 | 2 |
| Ingredient | Ingredient ratio (% by weight) | |
| Acetylsalicylic acid | 5.0 | 5.0 |
| Polysorbate 80 | 5.0 | |
| Diisopropyl adipate | | 5.0 |
| White petrolatum | 90.0 | |
| Hydrocarbon gel | | 90.0 |

### Examples 3 and 4 (creams)

According to the formulation shown in Table 2, after a solid base being melted on a water bath, thereto was added acetylsalicylic acid which was dissolved or dispersed in a solvent. Thereto was added an aqueous base which was dissolved in water and warmed. The mixture was blended until it became homogenous to prepare creams.

**Table 2**

| Formulation of cream containing acetylsalicylic acid | | |
|---|---|---|
| Example | 3 | 4 |
| Ingredient | Ingredient ratio (% by weight) | |
| Acetylsalicylic acid | 0.2 | 2.0 |
| Crotamiton | 5.0 | |
| Sesame oil | | 5.0 |
| Cetyl alcohol | 9.0 | 9.0 |
| White petrolatum | 8.0 | 8.0 |
| Hexyl decanol | 1.0 | 1.0 |
| Polyethylene glycol monostearate | 2.0 | 2.0 |
| Polyoxy (9) lauryl ether | 2.8 | 2.8 |
| Polyoxy (23) cetyl ether | 2.0 | 2.0 |
| Propylene glycol | 12.0 | 12.0 |
| Methylparaben | 0.1 | 0.1 |
| Propylparaben | 0.1 | 0.1 |
| Purified water | 57.8 | 56.0 |

### Example 5 (cataplasms)

According to the formulation shown in Table 3, a tackifier and a thickening agent were dissolved in a polyhydric alcohol warmed. After being cooled, thereto were added acetylsalicylic acid and other additives, and the mixture was homogenously blended. A crosslinking agent was added thereto to prepare an adhesive gel base. The gel base was spread on a support such as an unwoven fabric and cut in a desired size to prepare cataplasms.

**Table 3**

| Formulation cataplasm containing acetylsalicylic acid | |
|---|---|
| Example | 5 |
| Ingredient | Ingredient ration (% by weight) |
| Acetylsalicylic acid | 10.5 |
| Polyacrylic acid | 8.0 |
| Sodium polyacrylate | 4.0 |
| Sodium carboxymethylcellulose | 5.0 |
| Tartaric acid | 1.6 |
| Dihydroxy alminiumaminoacetate | 0.07 |
| Glycerin | 25.0 |
| Crotamiton | 2.0 |
| Castor oil | 1.0 |
| Purified water | 43.33 |

### Example 6 (powders)

According to the formulation shown in Table 4, potato starch, zinc oxide and acetylsalicylic acid were blended well until the mixture became homogenous to prepare powders.

**Table 4**

| Formulation of powder containing acetylsalicylic acid | |
|---|---|
| Example | 6 |
| Ingredient | Ingredient ratio (% by weight) |
| Acetylsalicylic acid | 0.05 |
| Potato starch | 95.95 |
| Zinc oxide | 4.00 |

### Comparative examples 1 to 3

According to the formulation shown in Table 5, vidarabine (adenine arabinoside: antiviral agent) was homogenously blended in white petrolatum to prepare ointments, and poviton iodine was dissolved in purified water to prepare solutions.

**Table 5**

| Comparative example | | | |
|---|---|---|---|
| Comparative example | 1 | 2 | 3 |
| Vidarabine | 3.0 | | |
| Povidone iodine | | 0.4 | 10.0 |
| White petrolatum | 97.0 | | |
| Purified water | | 99.6 | 90.0 |

### Test example

By administering an external preparation of the present invention containing aspirin, to patients (volunteers) having the viral infection injury symptoms such as vesicle, sore or ulcer caused by an infection of varicella-zostervirus, herpes simplex virus or enterovirusinfection accompanied with pain and pruritus at said affected parts, the effect was evaluated.

Improvement on a viral infection injury such as vesicle, sore or ulcer, and a pain and pruritus was evaluated by following five step-standard:
A: remarkably effective, B: effective, C: slightly effective, D: no change and E: worse.

In case of slightly effective or more than slightly effective, the cases were judged to be effective and the effectiveness was calculated.

### Test example 1

### Improvement of pain and prutitus associated with skin or mucosal injury caused by an infection of varicella·zostervirus, herpes simplex virus or enterovirus

To the affected part on patients (total 32 patients) having a viral infection injury such as vesicle, sore, or ulcer caused by an infection of varicella-zostervirus, herpes simplex virus or enterovirus associated with pruritus and pain, external preparation containing aspirin was administered and improvement on pruritus and pain was evaluated. Furthermore, as controls, an ointment containing an active ingredient which is used for such symptoms (Comparative example 1) and a disinfections used for treatment of mucosal injury symptom (Comparative examples 2 and 3) were administered and the improvement on them was evaluated as well.

The result was shown in Table 6.

**Table 6**

| Improvement on pain and pruritus at the affected part of a viral infection injury such as vesicle, sore or ulcer caused by infection of varicella· zostervirus, herpes simplex virus or enterovirus | | | | | | | | |
|---|---|---|---|---|---|---|---|---|
| Group | Drug (% by weight) | Number of patient | Evaluation | | | | | Effective rate(%) |
| | | | A | B | C | D | E | |
| Ointment base | - | 2 | 0 | 0 | 0 | 1 | 1 | 0 |
| Example 1 | Aspirin | 8 | 2 | 2 | 3 | 1 | 0 | 87.5 |
| Example 4 | Aspirin 2.0 | 5 | 0 | 2 | 2 | 1 | 0 | 80.0 |
| Example 5 | Aspirin 10.0 | 3 | 1 | 0 | 1 | 1 | 0 | 66.7 |
| Comparative example 1 | Vidarabine 3.0 | 8 | 1 | 2 | 3 | 2 | 0 | 62.5 |
| Comparative example 2 | Povidone iodine 0.4 | 4 | 0 | 0 | 1 | 2 | 1 | 25.0 |
| Comparative example 3 | Povidone iodine 10.0 | 2 | 0 | 0 | 0 | 1 | 1 | 0 |

As shown in the result on Table 6, Examples 1, 4 and 5 containing aspirin, comparing with Comparative example 1, more or equally inhibited pain and pruritus at the affected parts of a viral infection injury symptom such as vesicle, sore or ulcer caused by an infection of varicella-zostervirus, herpes simplex virus or enterovirus. On the other hand, Comparative examples 2 and 3 hardly showed the inhibition on pain and pruritus.

### Test example 2

### Improvement on a viral infection injury symptom such as vesicle, sore or ulcer caused by infection of varicella·zostervirus, herpes simplex virus or enterovirus

To the affected parts of patients (total 35 patients) having a viral infection injury symptom such as vesicle, sore or ulcer caused by infection of varicella·zostervirus, herpes simplex virus or enterovirus, an external preparation containing aspirin was administered, and the improvement of a viral infection injury symptom such as vesicle, sore or ulcer caused by infection of varicella-zostervirus, herpes simplex virus or enterovirus was evaluated. Furthermore, as controls, an ointment containing an active ingredient which is used for treatment such symptoms (Comparative example 1) and a disinfections used for treatment of the mucosal injury symptoms (Comparative example 2 and 3) were administered and the improvement on them was evaluated as well.

The result was shown in Table 7.

**Table 7**

| Improvement on patients having a viral infection injury symptom such as vesicle, sore or ulcer caused by infection of varicella·zostervirus, herpes simplex virus or enterovirus | | | | | | | | |
|---|---|---|---|---|---|---|---|---|
| Group Group | Drug (% by weight) patient | Number of | Evaluation | | | | | Effective rate (%) |
| | | | A | B | C | D | E | |
| Ointment base | - | 3 | 0 | 0 | 1 | 2 | 0 | 33.3 |
| Example 2 | Aspirin 5.0 | 8 | 2 | 3 | 3 | 0 | 0 | 100.0 |
| Example 3 | Aspirin 0.2 | 6 | 0 | 3 | 2 | 0 | 1 | 83.3 |
| Example 6 | Aspirin 0.05 | 4 | 1 | 1 | 1 | 1 | 0 | 75.0 |
| Comparative example 1 | Vidarabine 3.0 | 8 | 1 | 1 | 4 | 2 | 0 | 75.0 |
| Comparative example 2 | Povidone iodine 0.4 | 4 | 0 | 0 | 2 | 0 | 2 | 50.0 |
| Comparative example 3 | Povidone iodine 10.0 | 2 | 0 | 0 | 1 | 0 | 1 | 50.0 |

As shown in the result on Table 7, Examples 2, 3 and 6 containing aspirin, comparing with Comparative example 1, more or equally showed the therapeutic effect on a viral infection injury symptom such as vesicle, sore or ulcer caused by an infection of varicella-zostervirus, herpes simplex virus or enterovirus. On the other hand, Comparative examples 2 and 3 hardly showed fur inferior effect on them.

### INDUSTRIAL APPLICABILITY

By application of the external preparation of the present invention, with few side effects and without retardation of healing of a viral infection injury symptom, improvement on a viral infection injury symptom such as vesicle, sore or ulcer caused by a viral infection of varicella-zostervirus, herpes simplex virus or enterovirus, and inhibition of pain and pruritus at these affected parts became possible.

## Claims

1. An external preparation containing acetylsalicylic acid or a pharmacologically acceptable salt thereof as an active ingredient, which is used for treatment of skin or mucosal injury caused by a viral infection, together with an inhibition effect on a pain and pruritus at said affected part.

2. An external preparation containing acetylsalicylic acid or a pharmacologically acceptable salt thereof as an active ingredient, which is used for treatment of vesicle, sore or ulcer of skin and mucosa caused by a viral infection together with an inhibition effect on a pain or pruritus at said affected part.

3. The external preparation according to claim 1 or 2, wherein the viral infection is due to valicella-zostervirus, herpes simplex virus or enterovirus.

4. An excellent external preparation containing acetylsalicylic acid, or a pharmacologically acceptable salt thereof as an active ingredient, which is used for treatment of a pain or pruritus at an affected part on skin or mucosal injury caused by a viral infection.

5. An excellent external preparation containing acetylsalicylic acid, or a pharmacologically acceptable salt thereof as an active ingredient, which is used for treatment of a pain or pruritus at an part of vesicle, sore, or ulcer of skin or mucosa caused by a virus infection.

6. The excellent external preparation according to claim 4 or 5 wherein the virus infection is due to valicella·zostervirus, herpes simplex virus or enterovirus.

7. The external preparation according to any one of claims 1 to 6 wherein the concentration of acetylsalicylic acid or a pharmacologically acceptable salt thereof is 0.05 to 80% by weight.

8. A method for treatment of skin or mucosal injury caused by a viral infection by administering an effective amount of acetylsalicylic acid or a pharmacologically acceptable salt thereof to said affected part of a patient.

9. A method for treatment of vesicle, sore or ulcer of skin or mucosa caused by a viral infection, administering an effective amount of an external preparation containing acetylsalicylic acid or a pharmacologically acceptable salt thereof to said affected part of a patient.

10. The method for treatment according to claim 8 or 9, wherein the viral infection is due to varicella·zostervirus, herpes simplex virus or enterovirus.

11. A method for treatment of a pain and pruritus at skin or mucosal injury caused by a viral infection, administering an effective amount of an external preparation containing acetylsalicylic acid or a pharmacologically acceptable salt thereof to said part of a patient.

12. The method for treatment according to claim 11, wherein the viral infection is due to varicella·zostervirus, herpes simplex virus or enterovirus.
